# EUROPEAN PATENT APPLICATION

(11) **EP 4 755 410 A1**
(43) Date of publication of application: **10.06.2026**
(21) Application number: 25751901.7
(22) Date of filing: 30.01.2025
(51) Int. Cl.: A61M 1/00

(54) **CATHETER**

(30) Priority: 05.02.2024 JP 2024015359
(71) Applicant: Tsukada Medical Research Co., Ltd., Tokyo 161-0034 (JP)
(72) Inventor: YOSHIDA, Jumpei, Tokyo 161-0034 (JP); TSUKADA, Manabu, Tokyo 161-0034 (JP)
(74) Representative: Grünecker Patent- und Rechtsanwälte PartG mbB
(86) International application number: PCT/JP2025/002996
(87) International publication number: WO 2025/169831

(57) **Abstract**

An object of the present invention is to provide a urethral catheter capable of being easily inserted into a bladder and unlikely to induce discomfort in a patient even upon contact with a bladder wall. In a catheter 100, a tip portion 30 is provided at a distal end of a tube portion 10, and the tip portion 30 includes a tapered portion 31 that becomes thinner toward a distal end 31a, a proximal end portion 32 that is attached to the tube portion 10, and a curved portion 33, which is longer than the tapered portion 31 and curves between the tapered portion 31 and the proximal end portion 32.

## Description

### TECHNICAL FIELD

The present invention relates to a catheter mainly for urination management.

### BACKGROUND ART

A catheter for management of urination in a patient with dysuria and spinal injuries during and after a surgical procedure has conventionally been known (see e.g., Patent Literature 1). This type of catheter may be self-inserted by the patient, and therefore, various enhancements have been suggested to improve the ease of insertion.

### CITATION LIST

### PATENT LITERATURE

PTL 1: International Publication No. WO2005/018714

### SUMMARY OF INVENTION

### TECHNICAL PROBLEM

However, a catheter according to the above conventional technique may be difficult to insert into the bladder of a male patient. Particularly, during catheter insertion for the male patient, it may occasionally be difficult to pass the catheter through bulbar urethra. The catheter, which is advanced straight through pendulous urethra, enters the bulbar urethra, and takes a steep upward turn toward the bladder. At this point, due to individual variations in the shape of the bulbar urethra, the distal end of the catheter may fail to bend well along the bulbar urethra and abut against the wall surface of the bulbar urethra, for example, depending on the curvature of the bulbar urethra. This insertion procedure may be performed by a medical practitioner such as a doctor or nurse or by the patient themselves, and in either case, when the catheter is inserted into the urethra, the distal end of the catheter cannot be visually confirmed to have reached the bulbar urethra or similar region; instead, insertion is performed by relying solely on tactile sensation.

Furthermore, if the catheter is forcibly inserted in a state where the catheter abuts against the bulbar urethra, there is a risk that a region against which the catheter abuts may expand like a bag and form a false urethra. Once the false urethra has formed, in the case of a general catheter, the distal end of the catheter tends to enter a resulting depression in the false urethra, so that it is more difficult for the distal end of the catheter to rise in the bulbar urethra.

Furthermore, a Tiemann tip type catheter with an angled distal end is commonly used and is thought to be intended for the treatment of urethral strictures caused by trauma or similar conditions. The Tiemann tip type catheter inserted in the urethra is inserted into the bladder while dilating and entering the narrowed and stiffened urethra. As a result, the distal end of the Tiemann tip type catheter is composed of a comparatively rigid member, and the distal end of the tip is short and narrow, while the base of the tip is conically formed to match the thickness of a tube portion. The Tiemann tip type catheter may be used in cases involving a false urethra; however, the angled distal end of the catheter often lodges in the depression of the false urethra, preventing the catheter from advancing through the bulbar urethra and reaching the bladder. In addition, the Tiemann tip type catheter, due to its particularly rigid distal end, is prone to causing urethral injury and bleeding complications.

Furthermore, to treat urethral strictures, a procedure is performed in which a metal urethral bougie and a guide wire are used to insert a balloon catheter, guided by the guide wire that has been advanced into the bladder. However, because the urethral bougie is made of rigid metal, there is a risk of injuring the urethra.

Therefore, a catheter product has been desired, featuring a catheter distal end that bends smoothly along the bulbar urethra while minimizing the risk of false urethra formation and allowing easier insertion of the catheter into the bladder, even when a false urethra is already present.

Furthermore, when the catheter is placed in the bladder, a balloon is inflated to retain the catheter in the bladder, and the distal end of the catheter, which includes a urine drainage opening, is placed to protrude beyond the upper part of the inflated balloon. In this state, when urine is discharged from the bladder and the bladder contracts, the protrusion at the distal end of the catheter may contact the bladder wall, causing the patient to experience discomfort. Endoscopic observation has revealed that this contact can lead to inflammation of the bladder wall. As a result, there has been a demand for a product that ensures patient comfort even when the bladder contracts and the protruding distal end of the catheter contacts the bladder wall. In addition, when the bladder contracts, a distal end hole and side holes for urination formed at the distal end may become embedded in bladder wall mucosa, potentially resulting in reduced urination efficiency or even urinary obstruction.

It is an object of the present invention to solve the problems associated with the above conventional technology and to provide a urethral catheter that can be easily inserted into a bladder and cause little discomfort to a patient even when it comes into contact with the bladder wall.

### SOLUTION TO PROBLEM

The present invention provides a catheter comprising tip portion provided at a distal end of the tube portion, wherein the tip portion includes a tapered portion that becomes thinner toward a distal end, a proximal end portion that is attached to the tube portion, and a curved portion, which is longer than the tapered portion and curves between the tapered portion and the proximal end portion.

In this case, the tip portion may be an elastic body. The curved portion may have a flattened cross-section that becomes thinner in a curvature direction. The curved portion may include a concave portion formed on an inner periphery side. The curved portion may include a concave portion formed on an outer periphery side. The concave portion may have a U-shape that flares toward an edge. The concave portion may include a notch formed to flare in a circular shape at a location away from the edge. The curved portion may include multiple concave portions formed on the inner periphery side and the outer periphery side. The concave portion closest to the tapered portion may be formed on the inner periphery. The tip portion may include a multi-layer structure in which a surface portion is stacked around a core portion. The surface portion may be formed of a material that is softer than the core portion. The proximal end portion may include a first side hole formed in fluid communication with the tube portion. The curved portion may include a second side hole formed in fluid communication with the tube portion. The curved portion may include a first end portion in the proximal end portion side and a second end portion in the tapered portion side that have an approximately equal cross-sectional area.

### ADVANTAGEOUS EFFECTS OF INVENTION

The present invention provides a urethral catheter that can be easily inserted into a bladder and causes little discomfort to a patient even when it comes into contact with a bladder wall.

### BRIEF DESCRIPTION OF DRAWINGS

[Fig. 1] Fig. 1 schematically shows placement of a balloon catheter according to the present embodiment in a bladder.
[Fig. 2] Fig. 2(a) shows a side view of a tip portion, and Fig. 2(b) shows a front view of the tip portion.
[Fig. 3] Fig. 3 schematically shows passage of the balloon catheter through a false urethra.
[Fig. 4] Fig. 4 schematically shows the tip portion in contact with a bladder wall.
[Fig. 5] Fig. 5(a) shows a side view of a tip portion according to a second embodiment, and Fig. 5(b) shows a front view of the tip portion according to the second embodiment.
[Fig. 6] Fig. 6(a) shows a side view of a tip portion according to a third embodiment, and Fig. 6(b) shows a front view of the tip portion according to the third embodiment.
[Fig. 7] Fig. 7(a) shows a side view of a tip portion according to a fourth embodiment, and Fig. 7(b) shows a front view of the tip portion according to the fourth embodiment.
[Fig. 8] Fig. 8(a) shows a side view of a tip portion according to a fifth embodiment, and Fig. 8(b) shows a front view of the tip portion according to the fifth embodiment.
[Fig. 9] Fig. 9 shows a side view of a tip portion according to a sixth embodiment.

### DESCRIPTION OF EMBODIMENTS

Hereinafter, preferred embodiments of the present invention will be described with reference to the drawings.

### (First Embodiment)

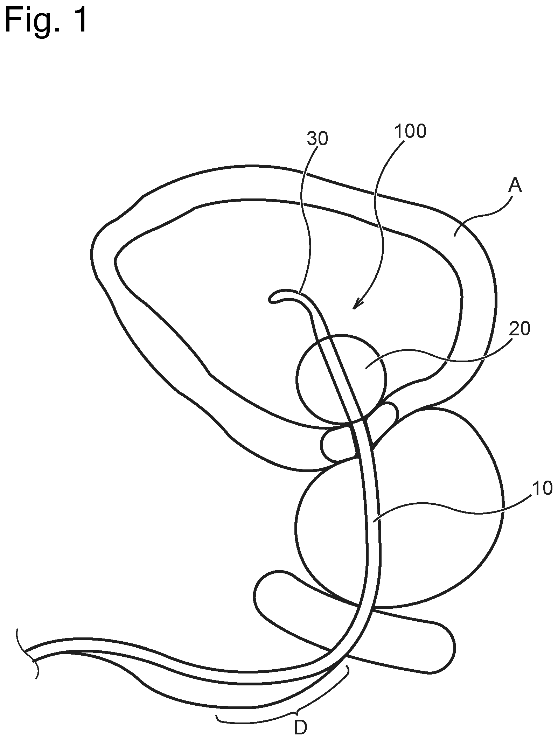

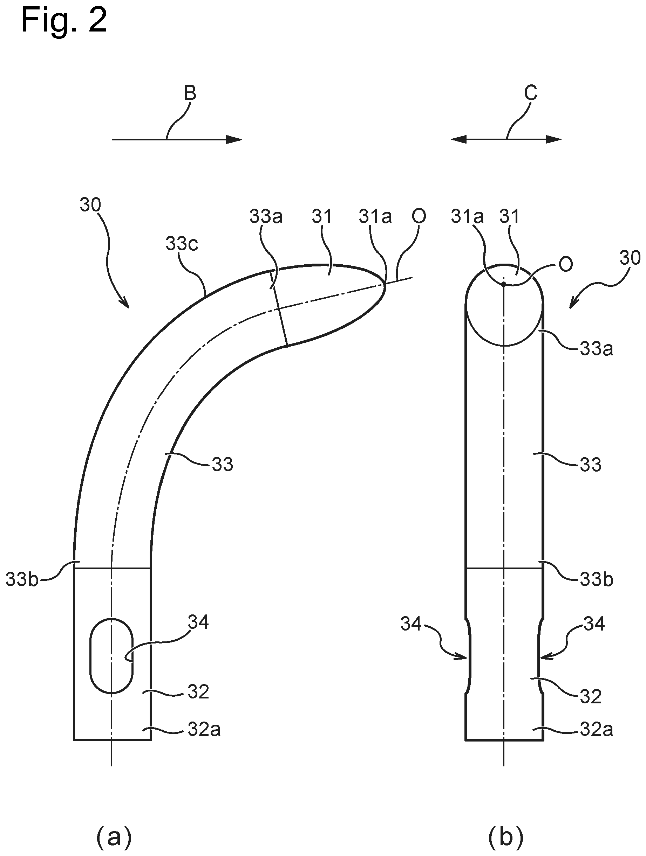

Fig. 1 schematically shows a balloon catheter placed in a bladder, Fig. 2(a) shows a side view of a tip portion, and Fig. 2(b) shows a front view of the tip portion.

A balloon catheter 100 includes a tube portion 10, a balloon portion 20, and a tip portion 30, as shown in Fig. 1.

The tube portion 10, the balloon portion 20, and the tip portion 30 are formed of silicone rubber, and may alternatively be formed of other materials such as latex, natural rubber, isoprene rubber, thermoplastic resins (e.g., PVC and urethane rubber), fluoro rubber, fluoro resin or the like.

The tube portion 10 penetrates the balloon portion 20, and the balloon portion 20 is provided on an outer periphery near a distal end by bonding. In other words, the balloon portion 20 is formed to surround the entire periphery of the tube portion 10. Furthermore, the tube portion 10 has a distal end to which the tip portion 30 is attached by bonding. In the tube portion 10, a urination lumen (not shown) for allowing urine to flow is formed along the extending direction of the tube portion 10. For the urination lumen, the urination lumen is in fluid communication with outside through a first side hole (opening) 34 (see Fig. 2) formed in the tip portion 30, and urine flowing through the first side hole 34 flows through the urination lumen to a branch portion (not shown) provided at the proximal end side of the balloon catheter 100.

In the tube portion 10, a balloon lumen (not shown) for inflating the balloon portion 20 is formed along the extending direction of the tube portion 10. The balloon lumen is in fluid communication in the balloon portion 20 near the distal end, and the balloon portion 20 is inflated by filling the balloon portion 20 with sterile water supplied from the branch portion at the proximal end side. As a result, as shown in Fig. 1, the balloon catheter 100 is placed by the balloon portion 20 inflated and lodged on a patient's bladder A, so that the catheter is prevented from being withdrawn.

The tip portion 30 is formed of an elastic material and is provided at the distal end of the tube portion 10. As shown in Fig. 2, the tip portion 30 includes a tapered portion 31 that becomes thinner toward the distal end, a proximal end portion 32 that is attached to the tube portion 10 (see Fig. 1), and a curved portion 33, which is longer than the tapered portion 31 and curves between the tapered portion 31 and the proximal end portion 32. These tapered portion 31, proximal end portion 32, and curved portion 33 are formed as a solid body.

The tapered portion 31 is formed such that a cross-section perpendicular to an axial center O does not expand from a distal end side end portion 33a of the curved portion 33, and the cross-section perpendicular to the axial center O becomes smaller toward the distal end. The outer peripheral surface of the tapered portion 31 is formed flush with the outer peripheral surface of the curved portion 33 at the distal end side end portion 33a. As a result, the tip portion 30 is formed by a continuous smooth surface from the curved portion 33 to the tapered portion 31.

The proximal end portion 32 is formed in a columnar shape so that its proximal end side is bonded to the tube portion 10. The proximal end portion 32 includes first side holes 34 and 34 formed at opposite ends in a width direction C perpendicular to a curvature direction B in which the curved portion 33 curves. This first side hole 34 is formed in an oblong shape and is in fluid communication with the urination lumen of the tube portion 10.

The outer peripheral surface of the proximal end portion 32 is formed flush with the outer peripheral surface of a proximal end side end portion 33b of the curved portion 33. As a result, the tip portion 30 is formed by a continuous smooth surface from the proximal end portion 32 to the curved portion 33. In other words, the tip portion 30 is formed by a continuous smooth surface from a proximal end side end portion 32a of the proximal end portion 32 to a distal end 31a of the tapered portion 31.

Since the curved portion 33 is formed longer along the axial center O than the tapered portion 31, it has a larger radius of curvature and is gently curved. This curved portion 33 is formed such that the cross-sectional areas of the proximal end side end portion 33b and the distal end side end portion 33a, taken perpendicular to the axial center O, are approximately equal. The curved portion 33 has a circular cross-section perpendicular to the axial center O, and this circular cross-section maintains a substantially constant cross-sectional area as it extends from the proximal end side end portion 33b to the distal end side end portion 33a.

For the tip portion 30 that is an elastic body, when the balloon catheter 100 is inserted into the bladder A through the urethra, the curved portion 33 flexes and straightens depending on the shape of the urethra, or returns to a curved state due to elastic force.

More specifically, in a straight pendulum urethra, the curved portion 33 flexes to conform to the straight pendulum urethra, allowing the tip portion 30 to pass through the pendulum urethra.

After passing through the pendulum urethra, the tip portion 30 proceeds through a curved bulbar urethra D, during which the curved portion 33 elastically returns to its original curved shape.

After passing through the bulbar urethra D, the tip portion 30 straightens as the curved portion 33 flexes, allowing the tip portion to advance sharply upward along the urethra. Once the tip portion reaches the bladder A and is no longer in contact with the urethra, the curved portion 33 elastically returns to its original curved shape. When urine in the bladder A is discharged and the bladder A contracts, the bladder wall may contact the tip portion 30. However, the tip portion 30 is designed to be flexible, as the curved portion 33 can bend further beyond its original curvature, thereby preventing excessive pressure against the bladder wall.

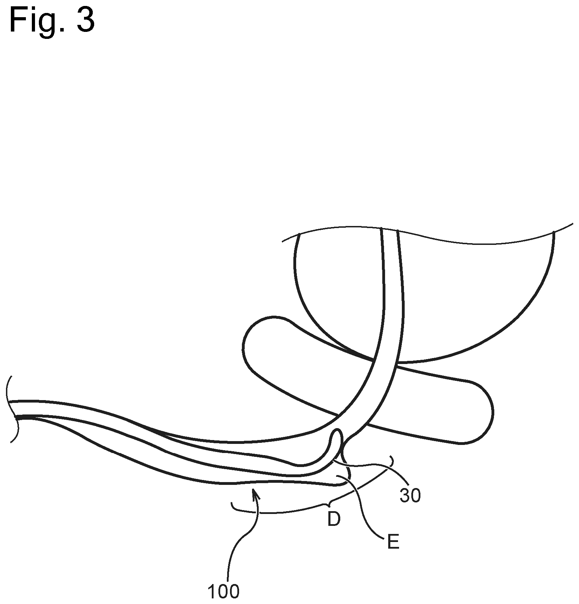

Fig. 3 schematically shows passage of a balloon catheter through a false urethra.

As shown in Fig. 3, even when inserted into the bladder A of a male patient with a false urethra E in the bulbar urethra D, the balloon catheter 100 is less likely to become caught in the false urethra E due to its appropriately curved tip portion 30. As a result, the balloon catheter 100 is inserted while bending well along the bulbar urethra D, avoiding the false urethra E.

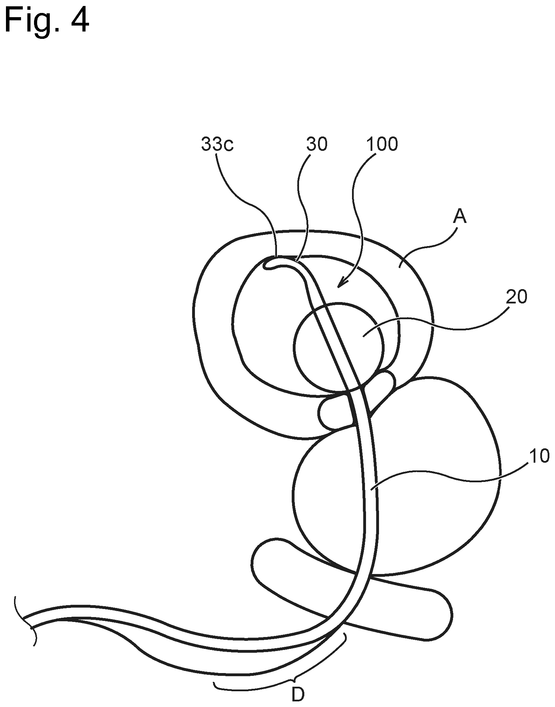

Fig. 4 schematically shows the tip portion in contact with the bladder wall.

The balloon catheter 100, which is placed in the bladder A by the inflated balloon portion 20, includes the tip portion 30 protruding from the balloon portion 20 as shown in Fig. 4. When urine is discharged from the bladder A and the bladder A contracts, the balloon catheter 100 comes into gentle contact with the bladder wall via a broad surface formed by the tapered portion 31 of the tip portion 30 and an outer peripheral side edge portion 33c of the curved portion 33.

The balloon catheter 100 according to the present embodiment includes the tip portion 30 provided at the distal end of the tube portion, wherein the tip portion 30 includes the tapered portion 31 that becomes thinner toward the distal end 31a, the proximal end portion 32 that is attached to the tube portion 10, and the curved portion 33, which is longer than the tapered portion 31 and curves between the tapered portion 31 and the proximal end portion 32. This allows the tip portion 30 to have a gentle curvature with a large radius of curvature. As a result, the balloon catheter 100 can be easily inserted into the bladder A and causes little discomfort to a patient even when it comes into contact with the bladder wall.

### (Second Embodiment)

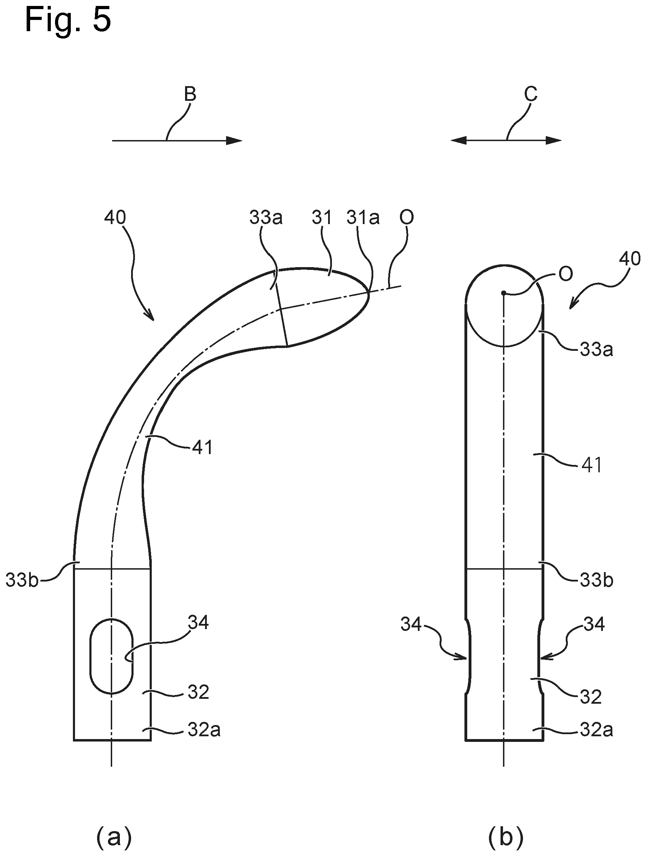

Fig. 5(a) shows a side view of a tip portion according to a second embodiment, and Fig. 5(b) shows a front view of the tip portion according to the second embodiment.

Hereinafter, the second embodiment will be described by using the same reference numerals for members that are substantially identical to those in the first embodiment.

As shown in Fig. 5(a), in a tip portion 40 according to the second embodiment, a curved portion 41 is different from the curved portion 33 (see Fig. 2) according to the first embodiment.

The curved portion 41 has a flattened cross-section that becomes thinner in the curvature direction. The curved portion 41 is formed in a circular shape such that cross-sections at a proximal end side end portion 33b and a distal end side end portion 33a, perpendicular to an axial center O, have approximately equal areas. On the other hand, the curved portion 41 is formed to change to a flattened cross-section, that is, an elliptic cross-section as it approaches an intermediate position between the proximal end side end portion 33b and the distal end side end portion 33a. Here, as shown in Fig. 5(b), the curved portion 41 is formed to maintain a substantially constant width along a width direction C, extending from the proximal end side end portion 33b to the distal end side end portion 33a.

### (Third Embodiment)

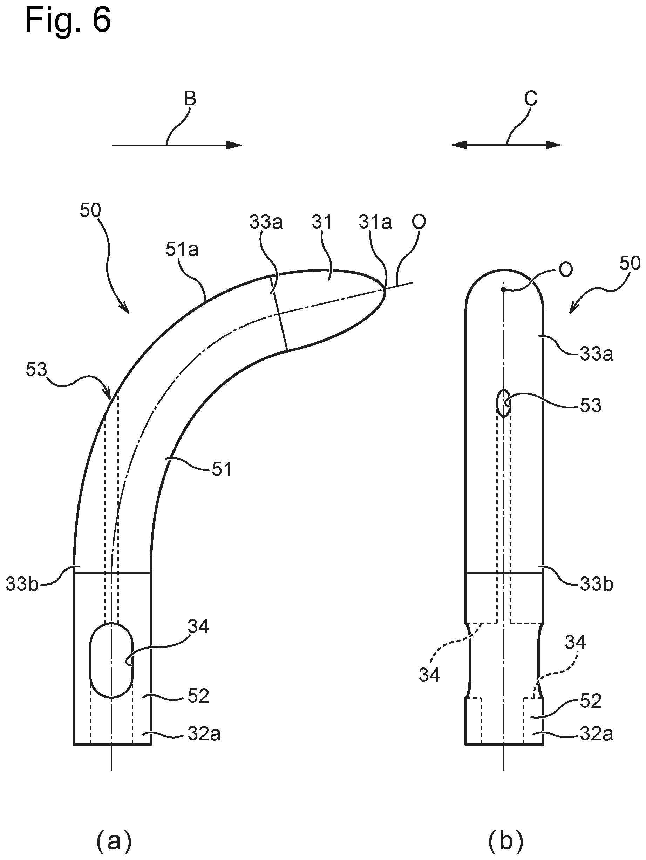

Fig. 6(a) shows a side view of a tip portion according to a third embodiment, and Fig. 6(b) shows a back view of the tip portion according to the third embodiment.

Hereinafter, the third embodiment will be described by using the same reference numerals for members that are substantially identical to those in the first embodiment.

In a tip portion 50 according to the third embodiment, as shown in Fig. 6(a), a curved portion 51 and a proximal end portion 52 are different from the proximal end portion 32 (see FIG. 2) and the curved portion 33 (see FIG. 2) according to the first embodiment.

In the curved portion 51, a second side hole 53 is formed at an outer peripheral side edge portion adjacent to an intermediate position between a proximal end side end portion 33b and a distal end side end portion 33a. This second side hole 53 penetrates the curved portion 51 and the proximal end portion 52 and is in fluid communication with a urination lumen of the tube portion 10 (see Fig. 1). The second side hole 53 penetrates through the proximal end portion 52 on an axial center O and is also in fluid communication with a pair of first side holes 34 and 34 that penetrate the proximal end portion 52 along a width direction C.

### (Fourth Embodiment)

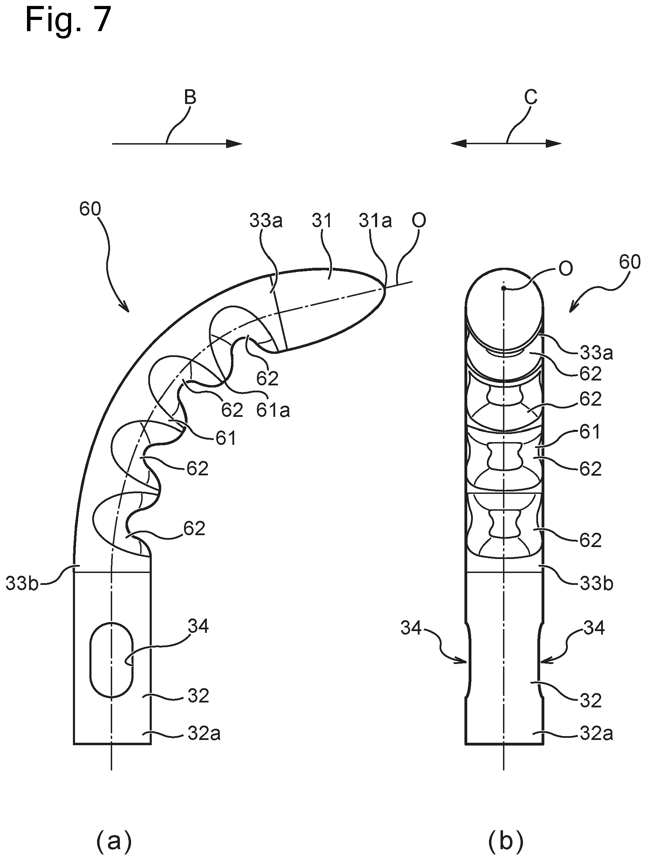

Fig. 7(a) shows a side view of a tip portion according to a fourth embodiment, and Fig. 7(b) shows a front view of the tip portion according to the fourth embodiment.

Hereinafter, the fourth embodiment will be described by using the same reference numerals for members that are substantially identical to those in the first embodiment.

In a tip portion 60 according to the fourth embodiment, as shown in Fig. 7(a), a curved portion 61 is different from the curved portion 33 (see Fig. 2) according to the first embodiment.

The curved portion 61 is formed by four concave portions 62 arranged in a row at an inner peripheral side edge portion 61a. Each of the concave portions 62 has a U-shape that flares toward the inner peripheral side edge portion 61a and is configured to retain a lubricant, such as lubricating jelly, to facilitate smooth insertion of the catheter into the urethra.

### (Fifth Embodiment)

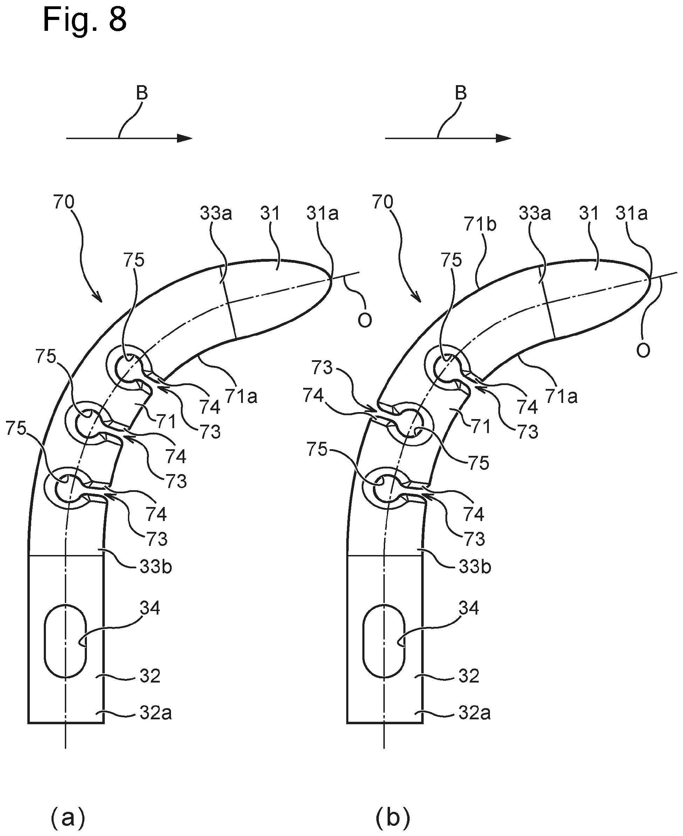

Fig. 8(a) shows a side view of a tip portion according to a fifth embodiment, and Fig. 8(b) shows a side view of a tip portion according to a modification of the fifth embodiment.

Hereinafter, the fifth embodiment will be described by using the same reference numerals for members that are substantially identical to those in the first embodiment.

As shown in Fig. 8(a), in a tip portion 70 according to the fifth embodiment, a curved portion 71 is different from the curved portion 33 (see Fig. 2) according to the first embodiment.

The curved portion 71 is formed by three concave portions 73 arranged in a row at an inner peripheral side edge portion 71a. Each of the concave portions 73 comprises a notch 74 that is cut from the inner peripheral side edge portion 71a, expands in a circular shape at a location away from the edge and communicates with a circular portion 75 of the circular shape. Each of the concave portions 73 is designed to retain a lubricant, such as lubricating jelly, to facilitate smooth insertion of the catheter into the urethra.

Furthermore, as shown in Fig. 8(b), the concave portions 73 may be formed on both the inner peripheral side edge portion 71a side and an outer peripheral side edge portion 71b side, and in this drawing, the concave portions are alternately formed on both the inner peripheral side edge portion 71a side and the outer peripheral side edge portion 71b side. The concave portion 73 that is closest to a tapered portion 31 is formed at the inner peripheral side edge portion 71a. When the catheter is placed in the bladder A, the concave portion 73 is less likely to contact the bladder wall, thereby allowing a broader surface area of the catheter to contact the bladder wall.

### (Sixth Embodiment)

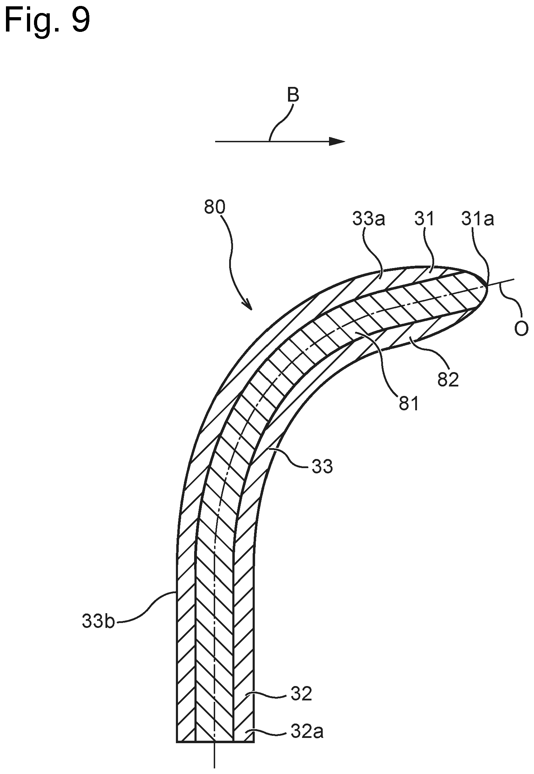

Fig. 9 shows a side view of a tip portion according to a sixth embodiment.

Hereinafter, the sixth embodiment will be described by using the same reference numerals for members that are substantially identical to those in the first embodiment.

As shown in Fig. 9, a tip portion 80 according to the sixth embodiment includes a multi-layer structure in which a surface portion 82 is stacked around a core portion 81. The surface portion 82 is formed of a material that is softer than the core portion 81. As a result, when the tip portion 80 comes into contact with the bladder wall, it touches the bladder wall with the soft surface portion 82, thereby making it less likely for the user to feel discomfort even upon contact with the bladder wall.

As above, the present invention has been described based on the embodiments, and the present invention is not limited to these embodiments. For example, in the above embodiment, the first side hole 34 is formed in the proximal end portion 32 and is not limited. If urine in the bladder A can be guided into the urination lumen, a side hole may be formed in the tube portion 10 without forming the first side hole 34 in the proximal end portion 32.

Furthermore, in the above embodiment, the concave portions 62 and 73 are mainly formed on the inner periphery side and are not limited. If the tip portion can be easily curved, the concave portion may be formed on the outer periphery side.

Furthermore, in the third embodiment, the second side hole 53 is formed at the position that penetrates the curved portion 51 straight from the proximal end portion 52 and is not limited. If the second side hole is in fluid communication with the urination lumen of the tube portion 10, the second side hole may be formed at a different location, such as on the side of the curved portion 51.

### REFERENCE SIGNS LIST

- 10: tube portion
- 20: balloon portion
- 30: tip portion
- 31: tapered portion
- 31a: distal end
- 32: proximal end portion
- 32a: proximal end side end portion
- 33: curved portion
- 33a: distal end side end portion
- 33b: proximal end side end portion
- 33c: outer peripheral side edge portion
- 34: first side hole
- 40: tip portion
- 41: curved portion
- 50: tip portion
- 51: curved portion
- 52: proximal end portion
- 53: second side hole
- 60: tip portion
- 61: curved portion
- 61a: inner peripheral side edge portion
- 62: concave portion
- 70: tip portion
- 71: curved portion
- 71a: inner peripheral side edge portion
- 71b: outer peripheral side edge portion
- 73: concave portion
- 74: notch
- 75: circular portion
- 80: tip portion
- 81: core portion
- 82: surface portion
- 100: balloon catheter
- A: bladder
- B: curvature direction
- C: width direction
- D: bulbar urethra
- E: false urethra
- O: axial center

## Claims

1. A catheter comprising:
a tip portion provided at a distal end of a tube portion,
wherein the tip portion includes a tapered portion that becomes thinner toward a distal end, a proximal end portion that is attached to the tube portion, and a curved portion, which is longer than the tapered portion and curves between the tapered portion and the proximal end portion.

2. The catheter according to claim 1, wherein the tip portion is an elastic body.

3. The catheter according to claim 1, wherein the curved portion has a flattened cross-section that becomes thinner in a curvature direction.

4. The catheter according to claim 1, wherein the curved portion includes a concave portion formed on an inner periphery side.

5. The catheter according to claim 1, wherein the curved portion includes a concave portion formed on an outer periphery side.

6. The catheter according to claim 4 or 5, wherein the concave portion has a U-shape that flares toward an edge.

7. The catheter according to claim 4 or 5, wherein the concave portion includes a notch formed to flare in a circular shape at a location away from an edge.

8. The catheter according to claim 1, wherein the curved portion include multiple concave portions formed on an inner periphery side and an outer periphery side.

9. The catheter according to claim 8, wherein the concave portion closest to the tapered portion is formed on the inner periphery.

10. The catheter according to claim 1, wherein the tip portion includes a multi-layer structure in which a surface portion is stacked around a core portion.

11. The catheter according to claim 10, wherein the surface portion is formed of a material that is softer than the core portion.

12. The catheter according to claim 1, wherein the proximal end portion includes a first side hole formed in fluid communication with the tube portion.

13. The catheter according to claim 1, wherein the curved portion includes a second side hole formed in fluid communication with the tube portion.

14. The catheter according to claim 1, wherein the curved portion includes a first end portion in the proximal end portion side and a second end portion in the tapered portion side that have an approximately equal cross-sectional area.
